# EUROPEAN PATENT SPECIFICATION

(11) **EP 2 394 680 B1**
(45) Date of publication and mention of the grant of the patent: **17.04.2013**
(21) Application number: 11165974.4
(22) Date of filing: 13.05.2011
(51) Int. Cl.: A61M 1/36

(54) **Blood container with improved functioning**
Blutbehälter mit verbesserter Funktion
Récipient pour sang doté d'un fonctionnement amélioré

(30) Priority: 09.06.2010 IT MI20101027
(43) Date of publication of application: 14.12.2011
(73) Proprietor: Eurosets S.r.l., 41036 Medolla (MO) (IT)
(72) Inventor: Ghelli, Nicola, 40018, San Pietro in Casale BO (IT); Costa Maianti, Edgardo, 41037, Mirandola MO (IT); Balanzoni, Roberto, 46020, San Giovanni Del Dosso MN (IT)
(74) Representative: Zoli, Filippo

(56) References cited:
- EP-A1- 2 108 392
- EP-A2- 0 333 119
- US-A- 5 744 047

## Description

The invention relates to a blood container with improved functioning.

It is known that, among the numerous apparatuses included in extracorporeal circuits through which blood is made to travel during some surgical operations, there are blood containers that are managed by operators in order to deal with particular situations that can arise.

Thus there are containers inserted on the line for venous return from the patient, termed "venous reservoir". If the containers are connected with a line for conveying the blood collected in the surgical field, the term is "cardiotomy reservoir".

Such containers are designed to receive a flow of blood which, during operation, assumes variable levels within a rather wide range, and moreover the containers comprise a filtering mass which the blood is made to cross, with the danger of blockage being particularly high in a cardiotomy reservoir designed to filter blood which, coming from the surgical field, brings with it grumes, clots and various impurities.

It thus happens that the blood, entering from above, floods the containers at the base and, with the progressing of the blockage situation in the filtering mass, or following an increase in the flow rate, the blood tends to raise its level in the containers, thus including an increasingly greater surface area in its passage.

This circumstance has strongly negative implications because, in proportion to the increase of the surface crossed by the blood, the degree to which the blood is traumatised rises.

EP 2 108 392 discloses a blood container comprising an external enclosure within which is provided a filtering wall which divides the space delimited by said enclosure into two portions.

The aim of the present invention is to provide a blood container the functioning of which is respectful of the characteristics of blood, thus optimally preserving the blood immunity from traumas.

Within this aim, an object of the invention is to provide an integrated container that comprises both venous reservoir and cardiotomy reservoir.

This aim and object are achieved by a blood container, according to the invention, characterised in that it comprises the features disclosed in the appended claims.

Further characteristics and advantages will become better apparent from the description of some preferred, but not exclusive, embodiments of the blood container according to the invention, illustrated by way of a nonlimiting example in the enclosed drawings wherein:
- Figure 1 is a longitudinal sectional view of the blood container according to the invention while not in the functioning condition;
- Figures 2 and 3 are views of two successive steps of the functioning of the blood container according to the invention;
- Figure 4 is a view of a first variation of the blood container according to the invention while not in the functioning condition;
- Figures 5 and 6 are views of two further variations of the blood container according to the invention while in a similar functioning condition thereof.

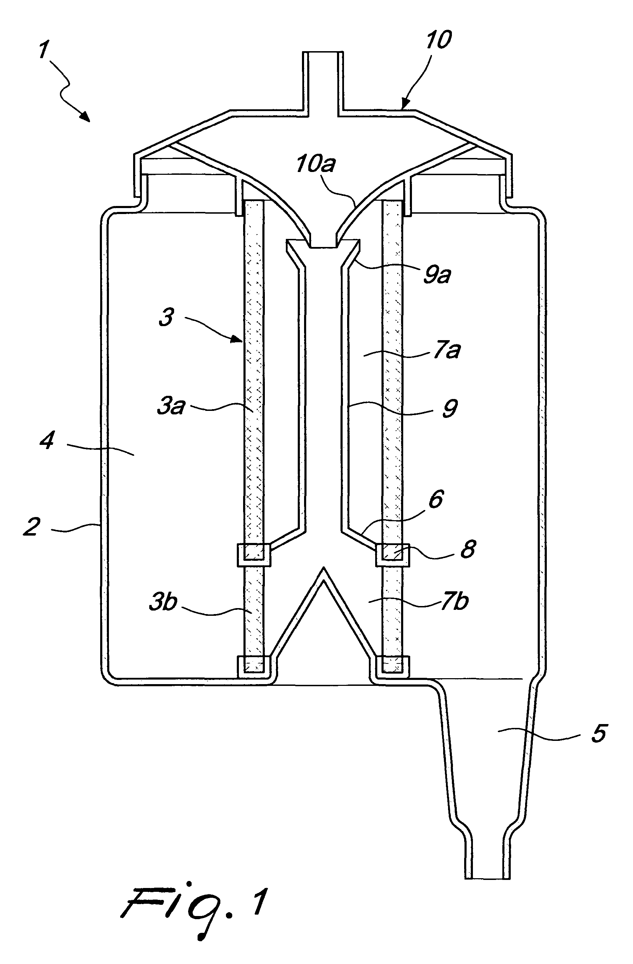

With reference to Figures 1 to 3, the reference numeral 1 generally designates a blood container comprising an external enclosure 2 which is provided internally with a filtering wall 3 that is made with known materials and divides the space delimited by the enclosure 2 into a portion 4 that is comprised between the enclosure and the wall 3 and is provided with a connector 5 for the outflow of the blood, and a portion that is comprised within the wall 3 and is provided with a partition 6 into two consecutive fractions: an overlying fraction 7a and an underlying fraction 7b which are delimited by corresponding sections 3a, 3b of the wall 3 and are conveniently separated by a resin ring 8, known as potting, precisely at the partition 6.

Within the overlying fraction 7a a duct 9 is provided for conveying to the underlying fraction 7b the blood received at an upper end 9a from an inlet connector 10, which with its appendage 10a enters the port that is open at the upper end 9a at the central region, while the peripheral region of this port is open onto the overlying fraction 7a.

The inlet connector 10 can be connected with a line for conveying the blood collected in the surgical field, and in this case the device performs the function of a cardiotomy reservoir. If, however, the connector is connected to the line for venous return from the patient, then the device is configured as a venous reservoir.

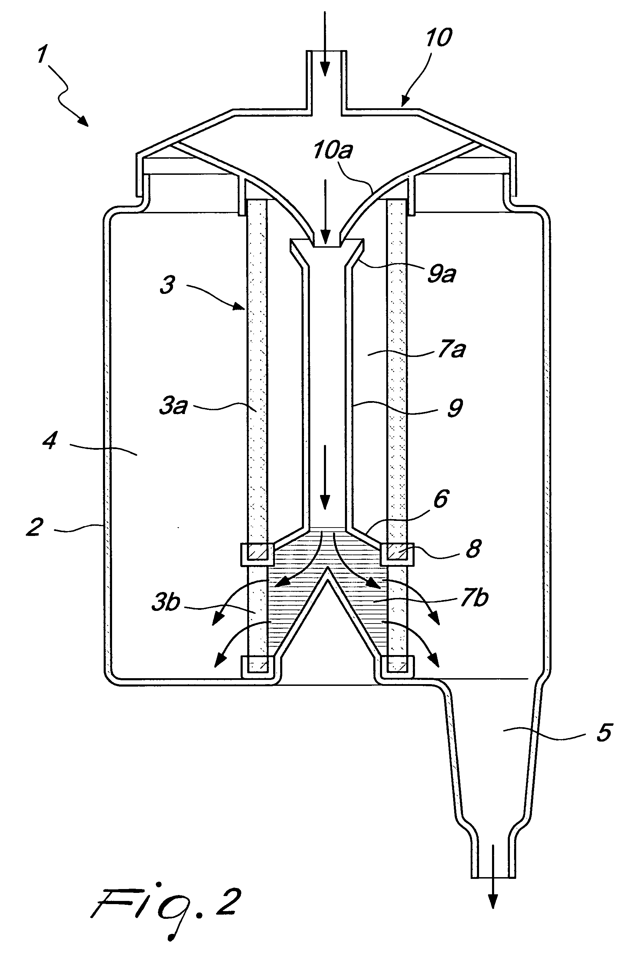

In the normal functioning of the blood container according to the invention, shown in Figure 2, the blood arrives at the underlying fraction 7b, exits therefrom and arrives, as shown by the arrows, at the portion of space 4, having been filtered by the section 3b of the wall 3 which has dimensions sufficiently reduced as to not cause effects of traumatisation.

At the first signs of blockage of the section 3b, or following an increase of the flow rate, the level of blood rises in the duct 9, thus forming a head that determines a hydrostatic pressure that is usually sufficient to still cause the blood to pass through the section 3b only.

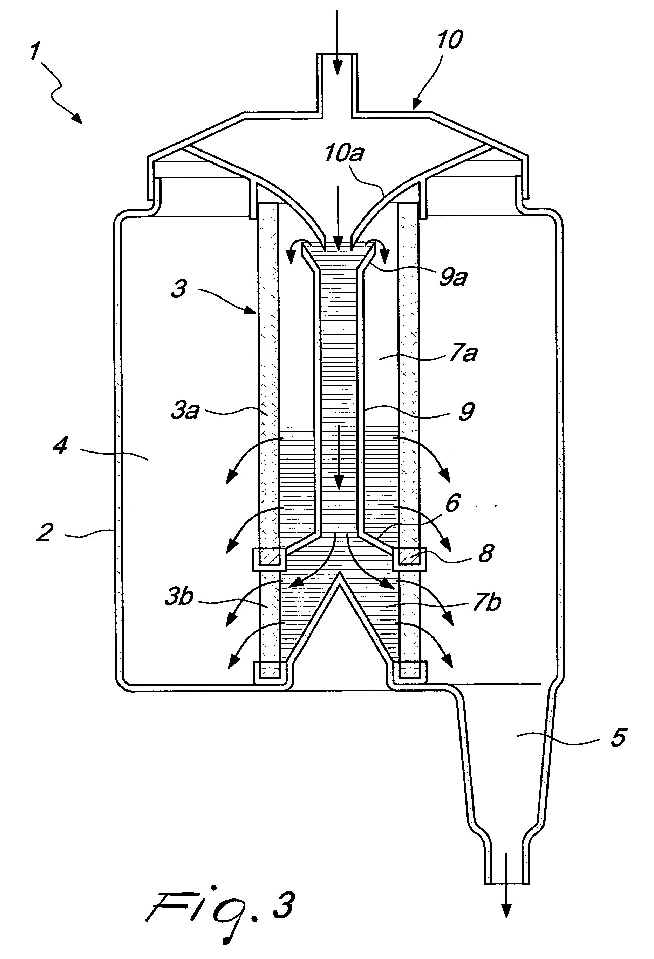

In the event of a blockage that is so strong, or in the event of an increase in flow rate that is so high, as to not allow the passage of the blood across the section 3b even with the aid of a head formed in the duct 9 up to the upper end 9a thereof, what will happen is simply the overflow, according to what is shown in Figure 3, of a portion of blood from the end 9a, with the falling thereof onto the base of the overlying fraction 7a and subsequent exit toward the portion of space 4 across a reduced band of the section 3 a of wall.

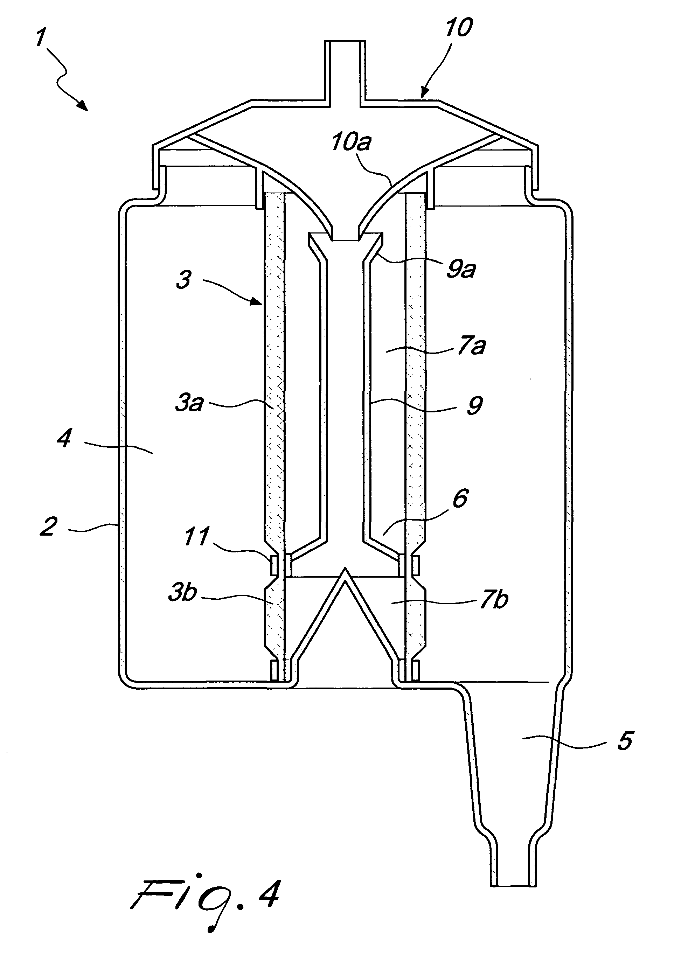

Figure 4 shows a variation of the blood container according to the invention in which nothing is changed with respect to the embodiment described, except the fact that the sections 3a, 3b of the filtering wall 3 are kept separate not by a ring of potting, but instead by a band 11.

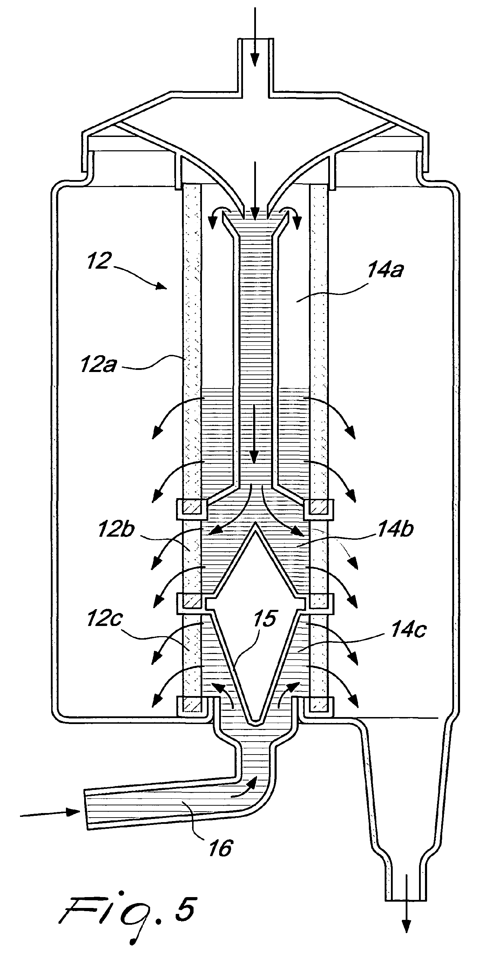

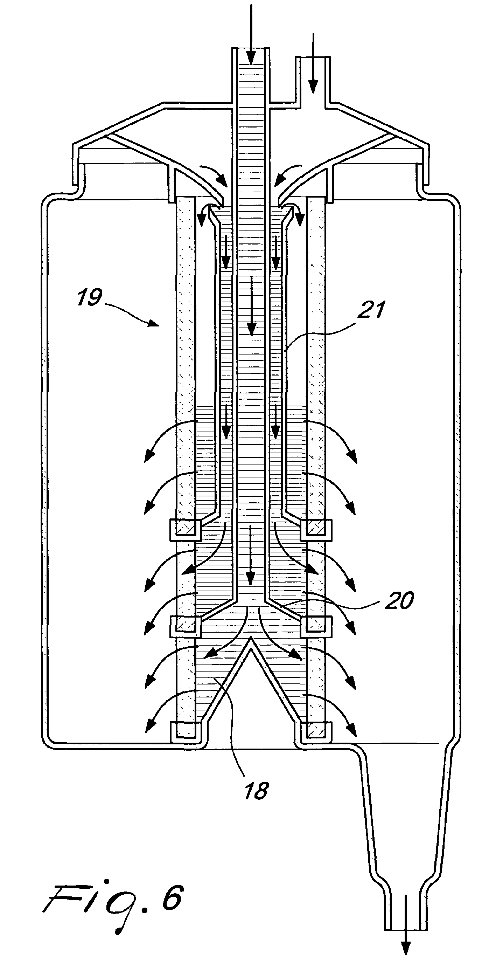

Figures 5 and 6 show two embodiments of an integrated container, which means a container comprising a venous reservoir and a cardiotomy reservoir in a single unit.

Hence Figure 5 shows how a filtering wall 12 which is comprised within an enclosure 13 is structured in such a way as to delimit a portion of space comprising fractions 14a and 14b, which are delimited by the separate sections of wall 12a, 12b, which perform the function of cardiotomy reservoir by exactly repeating the construction and functional characteristics of the container described previously, and comprising a further fraction 14c which is formed below the fractions 14a, 14b from which it is separated by means of a partition 15, which is delimited by a section of wall 12c which is suitably separated, and is provided at the bottom with a connector 16 which is designed to be connected with the line for venous return from the patient, thus acting as a venous reservoir.

The variation shown in Figure 6 differs from the integrated container of Figure 5 only in that a connector 17 for supplying venous blood return from the patient arrives at a corresponding fraction 18 which is comprised within the filtering wall 19 at a partition 20 thereof, extending coaxially with a duct 21 which conveys the blood collected in the surgical field.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A blood container, comprising an external enclosure (2) which is provided internally with a filtering wall (3), which divides the space delimited by said enclosure into a portion (4) that is comprised between said enclosure (2) and said filtering wall (3) and is provided with a connector (5) for the outflow of the blood, and a portion that is comprised within said filtering wall, **characterized in that** the portion that is comprised within said filtering wall is provided with a partition (6) for division into two consecutive fractions (7a, 7b), namely an overlying fraction (7a) and an underlying fraction (7b), which are delimited by corresponding separate sections (3a, 3b) of said filtering wall (3), within said overlying fraction (7a) there being a duct (9) for conveying to said underlying fraction (7b) blood received at its upper end (9a) from an inlet connector (10), which enters the port that is open at said end at the central region, whereas the peripheral region is open onto said overlying fraction (7a).

2. The device according to claim 1, **characterized in that** said blood inlet connector (10) is adapted to be connected to a line for conveying the blood collected in the surgical field.

3. The device according to claim 1, **characterized in that** said blood inlet connector (10) is adapted to be connected to the line for venous return from the patient.

4. The device according to claim 1, **characterized in that** it comprises, within the portion of space comprised within the filtering wall (12), an additional fraction (14c) of said portion which is separated by a corresponding partition (15) and is arranged below said underlying fraction (14b) that is designed to receive the blood collected in the surgical field, said additional fraction (140) having a connector (16) to the line for venous return from the patient.

5. The device according to claim 4, **characterized in that** the connector (16) to the line for venous return from the patient connects to said additional fraction (14c) at its bottom.

6. The device according to claim 4, **characterized in that** the connector (17) to the line for venous return from the patient connects to the additional fraction (18) at the partition (20).

## Patentansprüche

1. Blutbehälter, mit einem äußeren Gehäuse (2), das innen mit einer filternden Wand (3) versehen ist, die den von dem Gehäuse begrenzten Raum in einen Abschnitt (4), der zwischen dem Gehäuse (2) und der filternden Wand (3) gebildet wird und mit einem Anschluss (5) für den Abfluss des Blutes versehen ist, und einen Abschnitt, der innerhalb der filternden Wand gebildet wird, unterteilt, **dadurch gekennzeichnet, dass** der Abschnitt, der innerhalb der filternden Wand gebildet wird, mit einer Trennwand (6) zum Unterteilen in zwei aufeinanderfolgende Abteile (7a, 7b), und zwar in ein oben liegendes Abteil (7a) und ein unten liegendes Abteil (7b), die durch entsprechende getrennte Teile (3a, 3b) der filternden Wand (3) begrenzt werden, versehen ist, wobei innerhalb des oben liegenden Abteils (7a) ein Kanal (9) vorhanden ist, um an seinem oberen Ende (9a) aus einem Eintrittsanschluss (10) aufgenommenes Blut, das in die Öffnung eintritt, die an dem Ende in dem mittigen Bereich offen ist, während der Randbereich zum oben liegenden Abteil (7a) offen ist, zu dem unten liegenden Abteil (7b) zu befördern.

2. Vorrichtung nach Anspruch 1, **dadurch gekennzeichnet, dass** der Bluteintrittsanschluss (10) für das Anschließen an eine Leitung zum Befördern des im Operationsgebiet gesammelten Blutes angepasst ist.

3. Vorrichtung nach Anspruch 1, **dadurch gekennzeichnet, dass** der Bluteintrittsanschluss (10) für das Anschließen an die Leitung für venösen Rückstrom vom Patienten angepasst ist.

4. Vorrichtung nach Anspruch 1, **dadurch gekennzeichnet, dass** sie innerhalb des Raumabschnitts, der innerhalb der filternden Wand (12) gebildet wird, ein zusätzliches Abteil (14c) des Abschnitts aufweist, das durch eine entsprechende Trennwand (15) getrennt und unterhalb des unten liegenden Abteils (14b), das ausgestaltet ist, das im Operationsfeld gesammelte Blut aufzunehmen, angeordnet ist, wobei das zusätzliche Abteil (14c) einen Anschluss (16) an die Leitung für venösen Rückstrom vom Patienten besitzt.

5. Vorrichtung nach Anspruch 4, **dadurch gekennzeichnet, dass** der Anschluss (16) an die Leitung für venösen Rückstrom vom Patienten an das zusätzliche Abteil (14c) an dessen Unterseite anschließt.

6. Vorrichtung nach Anspruch 4, **dadurch gekennzeichnet, dass** der Anschluss (17) an die Leitung für venösen Rückstrom vom Patienten an das zusätzliche Abteil (18) an der Trennwand (20) anschließt.

## Revendications

1. Récipient pour du sang, comprenant une enveloppe externe (2) qui est dotée intérieurement d'une paroi filtrante (3), qui divise l'espace délimité par ladite enveloppe en une portion (4) qui est comprise entre ladite enveloppe (2) et ladite paroi filtrante (3) et est dotée d'un raccord (5) pour le flux de sortie du sang, et une portion qui est comprise à l'intérieur de ladite paroi filtrante, ***caractérisé en ce que*** la portion qui est comprise à l'intérieur de ladite paroi filtrante est dotée d'une cloison (6) de séparation en deux parties consécutives (7a, 7b), à savoir une partie sus-jacente (7a) et une partie sous-jacente (7b), qui sont délimitées par des sections séparées correspondantes (3a, 3b) de ladite paroi filtrante (3), un conduit (9) étant présent dans ladite partie sus-jacente (7a) pour acheminer jusqu'à ladite partie sous-jacente (7b) du sang reçu à son extrémité supérieure (9a) d'un raccord d'entrée (10) qui pénètre dans l'orifice qui est ouvert à ladite extrémité au niveau de la zone centrale, alors que la zone périphérique est ouverte sur ladite partie sus-jacente (7a).

2. Dispositif selon la revendication 1, ***caractérisé en ce que*** ledit raccord d'entrée (10) du sang est apte à être raccordé à une ligne destinée à acheminer le sang collecté dans le champ opératoire.

3. Dispositif selon la revendication 1, ***caractérisé en ce que*** ledit raccord d'entrée (10) du sang est apte à être raccordé à la ligne pour le retour veineux du patient.

4. Dispositif selon la revendication 1, ***caractérisé en ce qu'**il* comprend, dans la portion d'espace comprise à l'intérieur de la paroi filtrante (12), une partie supplémentaire (14c) de ladite portion qui est séparée par une cloison correspondante (15) et est disposée au-dessus de ladite partie sous-jacente (14b), qui est conçue pour recevoir le sang recueilli dans le champ opératoire, ladite partie supplémentaire (14c) étant dotée d'un raccord (16) avec la ligne pour le retour veineux du patient.

5. Dispositif selon la revendication 4, ***caractérisé en ce que*** le raccord (16) avec la ligne pour le retour veineux du patient est raccordé à ladite partie supplémentaire (14c) dans son fond.

6. Dispositif selon la revendication 4, ***caractérisé en ce que*** le raccord (17) avec la ligne pour le retour veineux du patient est raccordé à ladite partie supplémentaire (18) au niveau de la cloison (20).
